(19) **Europäisches Patentamt / European Patent Office / Office européen des brevets**

(11) **EP 3 620 788 B1**

(12) **EUROPEAN PATENT SPECIFICATION**

(45) Date of publication and mention
of the grant of the patent:
**08.05.2024 Bulletin 2024/19**

(21) Application number: **18794807.0**

(22) Date of filing: **28.04.2018**

(51) International Patent Classification (IPC):
**G01N 33/00** (2006.01)   **A24C 5/34** (2006.01)
**A24C 5/343** (2006.01)

(52) Cooperative Patent Classification (CPC):
**A24C 5/343; A24C 5/3406**

(86) International application number:
**PCT/CN2018/085078**

(87) International publication number:
**WO 2018/202005 (08.11.2018 Gazette 2018/45)**

(54) **AN APPARATUS FOR DETECTING HEAD FALLING TENDENCY PERFORMANCE OF A CIGARETTE BASED ON SIMULATING HUMAN BEHAVIOR FEATURES OF FLICKING ASH AND THE METHOD THEREOF**

VORRICHTUNG ZUR ERKENNUNG DER TENDENZ DES FALLENS EINES ZIGARETTENKOPFES AUF BASIS DER SIMULATION VON EIGENSCHAFTEN DES MENSCHLICHEN VERHALTENS BEIM SCHNIPPEN VON ASCHEN UND ENTSPRECHENDES VERFAHREN

DISPOSITIF DE DÉTECTION DE TENDANCE À LA CHUTE D'UNE TÊTE DE CIGARETTE BASÉ SUR LA SIMULATION DE CARACTÉRISTIQUES DE COMPORTEMENT HUMAIN LORSQUE LES CENDRES SONT TAPOTÉS ET PROCÉDÉ CORRESPONDANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.05.2017 CN 201710303000**

(43) Date of publication of application:
**11.03.2020 Bulletin 2020/11**

(73) Proprietors:
• **Zhengzhou Tobacco Research Institute of CNTC**
**Zhengzhou, Henan 450001 (CN)**
• **Hefei Institute Of Physical Science,**
**Chinese Academy of Sciences**
**Hefei, Anhui 230088 (CN)**
• **China Tobacco Jiangxi Industrial Co., Ltd.**
**Nanchang, Jiangxi 330096 (CN)**

(72) Inventors:
• **LI, Bin**
**Zhengzhou**
**Henan 450001 (CN)**
• **ZHANG, Yi**
**Nanchang**
**Jiangxi 330096 (CN)**

• **SANG, Yaoshuo**
**Hefei**
**Anhui 230088 (CN)**
• **ZHANG, Mingjian**
**Zhengzhou**
**Henan 450001 (CN)**
• **HONG, Liu**
**Nanchang**
**Jiangxi 330096 (CN)**
• **LI, Zhigang**
**Hefei**
**Anhui 230088 (CN)**
• **XU, Bingyang**
**Nanchang**
**Jiangxi 330096 (CN)**
• **XU, Zhenyu**
**Nanchang**
**Jiangxi 330096 (CN)**
• **TANG, Xiaoling**
**Nanchang**
**Jiangxi 330096 (CN)**
• **ZHANG, Long**
**Hefei**
**Anhui 230088 (CN)**

**(Cont. next page)**

(74) Representative: **Schiweck Weinzierl Koch**
**Patentanwälte Partnerschaft mbB**
**Ganghoferstraße 68 B**
**80339 München (DE)**

(56) References cited:
| | |
|---|---|
| **CN-A- 102 937 639** | **CN-A- 103 257 212** |
| **CN-A- 105 571 960** | **CN-A- 105 571 960** |
| **CN-A- 105 606 766** | **CN-A- 105 628 273** |
| **CN-A- 105 651 626** | **CN-A- 106 442 901** |
| **CN-A- 106 950 338** | **CN-A- 107 085 075** |
| **CN-U- 204 165 850** | **DE-C2- 19 854 008** |
| **KR-B1- 101 731 465** | |

**Description**

**FIELD OF THE INVENTION**

The invention belongs to the technical field of quality inspection of cigarette products, and particularly relates to a cigarette head falling tendency performance detecting apparatus based on simulating human behavior features of flicking ash and the method thereof.

**BACKGROUND OF THE INVENTION**

In recent years, market feedback caused by consumers' resentment caused by cigarette burning cones has occurred from time to time, and the range of brands involved has become more and more extensive, especially in high-grade cigarette products. In the process of cigarette consumption, the head falling of the burning cone will not only cause the cigarette loss to interrupted the smoking, but the falling butt may also burn clothing and furniture, and bring the risk of fire (Peace. World No Tobacco Day: Interpretation of the cigarette butt [J]. China Fire, 2011 (11): 38-42.). The issue of the cigarettes head falling has received more and more attention, and it is hoped to seek improvement through technological innovation (Wang Haibin, Liu Dehu, Wu Zhaogang, et al. Analysis and research on the burning phenomenon of cigarette burning end [A]. China Tobacco Society 2010 Symposium Set, 2010, 285-287.).

The main reason for the possible of the cigarette burning cone head being dropped is due to the matching of the burning rate of the cigarette paper with the tobacco, the filling of the tobacco in the cigarette paper, and the control of the axial distribution through the flat disk of the cigarette maker during the rolling process and so on. The measurement of the performance of the head falling is an important basis for the evaluating the effectiveness of various technical improvements. At present, the detection of the performance of the cigarette head falling can only rely on the consumer's conscious qualitative judgment in the process of cigarette smoking and flicking ashes, and the data is obtained by this method with low efficiency and poor repeatability.

The Chinese invention patent (CN 102937639 A) developed a cigarette head falling detection device by simulating flicking ashes of the consumer's habits, and determined whether the head of the burning cigarette was dropped by an intermittent tapping. The device uses a tapping method which is different from the actual consumer's habit, and the test results of the method are too simple to give an effective objective evaluation.

The cigarette falling head detecting device involved in the Chinese utility model patent (CN 204165850 U) has improved the problem in that the device's flicking force and angle cannot be flexibly changed in the Chinese invention patent (CN 102937639 A), but there are still some inadequacies. The flicking method disclosed in the patent still relies on external mechanical force to strike the cigarette, but such flicking simulation performed by the mechanical component with simple tapping action is only apparent, and the actual stress situation in the process is not considered in nature.

The Chinese invention patent (201310227468.X) is a device for detecting the tendency of cigarettes head-drop by rotation, and is characterized in that the device comprises a base, a motor, a cigarette holding mechanism driven by a motor, and a safety shield. The invention tests the falling phenomenon of the burning cone of the cigarette by the rotation method, and opens up a new method for detecting the burning cone, and uses the multiple detection to obtain the statistical data of the falling head, thereby obtaining the performance index of the tendency of the cigarette falling head when burning. The invention has the advantages in that the rotation time and the number of revolutions is controllable, different detection strengths can be applied for different samples, and the application range is wide; the detection method is simple and convenient, the time is short, and the detection cost is low. However, since there is no actual smoking process in this method, it does not meet the behavior of human knocking down ashes and flicking ashes, and there is a problem in evaluating the performance of the head falling in the actual state.

The Chinese invention patent (201510973214.1) proposes an automatic control detection device and detection method for the burning cone head falling of the cigarette, and designs the detection device by studying comparison between the mechanical behavior of the mechanics and human flicking ashes to ensure the mechanical behavior being consistent with human behavior when flicking cigarettes by the mechanical device, so as to provide a unified, objective and accurate detection method for the detection of cigarette head falling tendency. This method has not yet explained how to establish a cigarette head falling tendency detection method based on simulating the behavior of consumer flicking ashes, the behavioral characteristics of how consumers flicking ashes is the basic problem of the proposed and applied method of cigarette head falling performance evaluation, and it is more important to establish the detection method based on the simulated human flick ash behavior characteristics.

The Chinese invention patent CN105571960 A proposes a device for detecting the performance of the burning cone head falling of the cigarette during smoking, through mechanical analysis, it simulates the mechanical behavior of humans hitting and flicking cigarette, provides a device for detecting the performance of the burning cone falling head during the cigarette smoking process. Through this device, the performance of the burning cone falling head is determined. However, this performance detection device focuses on controlling the suction unit and does not conduct specific research on the behavior of cigarette ash dropping when flicking the cigarette. Therefore, it cannot accurately simulate the behavior of cigarette consumers when flicking, and cannot provide objective and accurate detection basis for the evaluation for the performance of the falling head of the cigarette.

## SUMMARY OF THE INVENTION

An object of the present invention is based on the deficiencies and problems in the above patents, and through the investigation of the characteristics of the smoked cigarette dropping behavior of the cigarette consumer, the data of the smoking ashes dropping behavior of the cigarette consumer is obtained. The cigarette head falling tendency performance detecting apparatus and method based on simulating human behavior features of flicking ashes of the cigarette is invented, and the invention provides an objective and accurate detection basis for the evaluation of the cigarette head falling performance.

An aspect of the present invention is to provide a cigarette head falling tendency performance detecting apparatus based on simulating human behavior features of flicking ash of a cigarette, comprising:

a clamping unit for clamping the cigarette;
a smoking unit, the smoking unit is connectable to one end of the cigarette at a contact surface to smoke the cigarette;
a flicking unit, the flicking unit being disposed adjacent to the clamping unit, and said flicking unit capable of flicking the cigarette; and
a control unit, the control unit being coupled with the smoking unit and the flicking unit, respectively, to control smoking and flicking actions;
wherein the flicking unit comprises:

a flicking arm; and
a flicking hammer, the flicking hammer is provided at one end of said flicking arm to flick the cigarette under the driving of the flicking arm, and when the flicking hammer is in contact with the cigarette, the angle between the flicking arm and the cigarette is between 30-60 degree.

In one embodiment, the clamping unit is clamped at a filter portion of the cigarette, and the one end is a free end of said filter portion.

In one embodiment, the flicking hammer is made of a material having a Mohs hardness of between 1 to 3.

In one embodiment, the flicking strength of the flicking hammer applied to the cigarette is between 0.196N to 0.588N (20gf to 60gf).

In one embodiment, the duration for every flicking applied by the flicking hammer is between 0.025 seconds to 0.035 seconds.

In one embodiment, the width of a flicking point at where the flicking hammer flicks the cigarette is between 9.5mm to 10.5mm, and a distance between said flicking point and said contact surface is 30mm to 32mm.

In one embodiment, the clamping unit is made of a material having Shore hardness between 0.4HA-5.0HA.

In one embodiment, a clamping width of the clamping unit clamping the cigarette is between 9.5mm-10.5mm, and a clamping strength of the clamping unit is between 0.156N-0.176N (16gf-18gf), and the distance from a clamping point of the clamping unit to said one end is between 18mm-20mm.

In one embodiment, the control unit is used for controlling a clamping strength of the clamping unit, a smoking strength and a smoking frequency of the smoking unit, and a flicking cycle, a location of the flicking point, and a flicking strength of the flicking unit.

Another aspect of the present invention is to provide a method for detecting a cigarette head falling tendency performance using the cigarette head falling tendency performance detecting apparatus, comprising:

step A: using the clamping unit to clamp the cigarette, and lighting the cigarette;
step B: the smoking unit is activated by the control unit to simulate a smoking action of human;
step C: taking k times dragging by the smoking unit as a cycle, and flicking unit is activated by the control unit to perform a round of flicking on the cigarette to simulate a human's flicking action;
step D: stopping the detection by the control unit when the cigarette's head is dropped or the cigarette burns to a predetermined test termination mark; and
step E: repeating the step B, the step C, and the step D for 40 cigarettes, and recording the number n of occurrences of the head drop so as to calculate the cigarette burning cone head falling tendency HCFP using the following formula:

$$HCFP = n\,/\,40 \times 100\%.$$

In one embodiment, the step A, the step B, the step C, the step D, and the step E are performed in a constant temperature and humidity environment.

In one embodiment, the clamping width of the clamping unit clamping the cigarette is between 9.5mm-10.5mm, and the clamping strength of the clamping unit is between 0.156N-0.176N (16gf-18gf), and the distance from a clamping point of the clamping unit to said one end is between 18mm-20mm.

In one embodiment, in the step C, the one round of flicking comprises performing 1-4 times of flicking on the cigarette using the bombing unit, and the interval time between adjacent two times of flicking is no more than 1 second.

In one embodiment, in step C, the flicking strength of the flicking hammer applied to the cigarette is between 0.196N and 0.588N (20gf and 60gf).

In one embodiment, the width of a flicking point at where the flicking hammer flicks the cigarette is between 9.5mm to 10.5mm, and a distance between said flicking point and said one end is 30mm to 32mm.

In one embodiment, two sets of tests are applied to each cigarette sample, and after the step E, the final HCFP of the cigarette is represented by the average value of the two sets of detection results, and when the absolute difference of the measurement results of two set of detection results is greater than 20%, the detection is performed again.

In the presenting invention, by investigating the behavior of flicking ash by consumers, regarding regular cigarettes and slim cigarettes sold in the market, taking the behavior of flicking the cigarette to apply the force to the ash as the force applying basis, the behavior characteristics data of when the smoker flicks the cigarette to cause ashes fallen down is obtained according to aspects of the characteristics of flicking cigarette to cause falling ash behavior, the characteristics of holding a cigarette, and the behavioral characteristic of force applying process, and based on such data (as shown in Table 1), the test operating conditions in the cigarette head falling tendency performance detecting method is formed. Therefore, an objective and accurate test basis for the evaluation of cigarette head falling performance is provided, and technical data support for related cigarette head falling research is provided as well.

## BRIEF DESCRIPTION OF THE DRAWINGS

Fig. 1 is a side view of a cigarette head falling tendency performance detecting apparatus based on simulating the behavior features of human flicking ash according to an exemplary embodiment of the present invention; and

Fig. 2 is a top view of the cigarette head falling tendency performance detecting apparatus based on simulating the behavior features of human flicking ash shown in Fig. 1.

Wherein: 1. a cigarette; 2. flicking angle (when the flicking arm moves until the horizontal plane is parallel, the angle is formed by the center line of the flicking arm in the top view and the symmetry plane of the cigarette); 3. a flicking arm; 4. a flicking hammer; 5. a cigarette holder; 6. a smoking unit; X1. clamping position; X2. clicking position; X3. a holder width; X4. a hammer width.

## DETAILED DESCRIPTION OF THE EMBODIMENTS

Specific embodiments of the present invention will be further described in detail with reference to the accompany drawings.

In the presenting invention, by investigating the behavior of flicking ash by consumers, regarding regular cigarettes and slim cigarettes sold in the market, taking the behavior of flicking the cigarette to apply the force to the ash as the force applying basis, the behavior characteristics data of when the smoker flicks the cigarette to cause ashes fallen down is obtained according to aspects of the characteristics of flicking cigarette to cause falling ash behavior, the characteristics of holding a cigarette, and the behavioral characteristic of force applying process, and based on such data (as shown in Table 1), the test operating conditions in the cigarette head falling tendency performance detecting method is formed.

Table 1: the test conditions for regular cigarettes and slim cigarettes determined under the behavior of flicking the cigarette to apply the force to cause ash falling (In the table below 1gf=0.0098N)

| Force applying mode | Behavior of flicking the cigarette to apply the force to cause ash falling | |
|---|---|---|
| Cigarette Type | Regular | Slim |
| Force Strength (gf) | 20gf -60gf | 20gf-60gf |
| Force Applying Time Length (s) | 0.03±0.005 | 0.03±0.005 |

(continued)

| Force applying mode | Behavior of flicking the cigarette to apply the force to cause ash falling | |
|---|---|---|
| Force Applying Position (mm) | 30±0.5 | 32±0.5 |
| Clamping Strength (gf) | 18±2 | 16±2 |
| Clamping Position (mm) | 18±0.5 | 19±0.5 |
| Force Applying Frequency | 2 | 1 |
| Force Applying Timing | Flicking action is applied after every drag of smoking completing after the second drag of smoking is taken | |
| Clamping width (mm) | 10 | |
| Hammer width (mm) | 10 | |
| Ending Time (mm) | 40±0.5 | 42±0.5 |
| Smoking Mode | ISO3308 Standard Smoking Mode | |

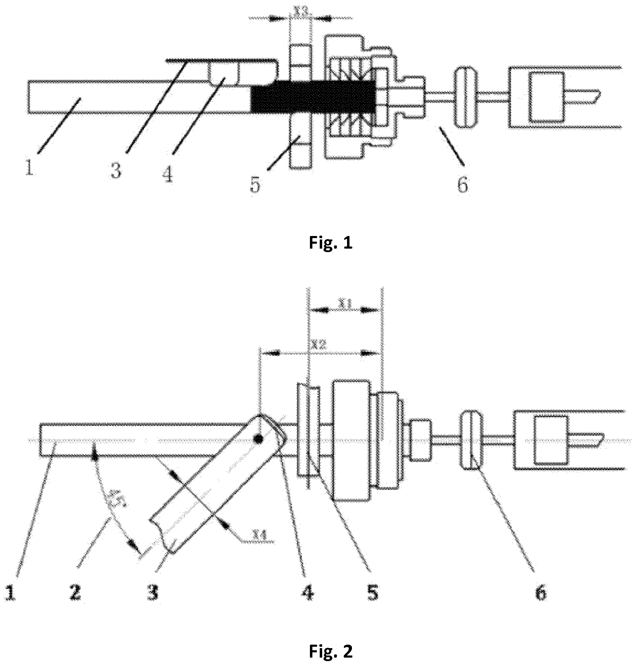

The invention provides a burning cone head falling tendency test device (see Figs. 1 and 2) in accordance with the operating conditions, the test device comprising a cigarette smoking unit, a clamping device unit and a flicking device unit, the specific conditions are as follows:

1. With a cigarette smoking unit in accordance with GB/T 16450

2. The flicking device unit requires that a Mohs hardness of the material of the flicking hammer 4 is in the range of 1-3, the width X4 of the hammer 4 conforms to (10 ± 0.5) mm, and the flicking angle 2 (the angle formed between the flicking arm 3 and the cigarette 1 the flicking arm 3 when the ash is flicked) can be adjusted from 30° to 60°, and the flicking strength (the strength of the flicking hammer 4 applied on the cigarette 1) should be adjustable within a range of (0.196-0.588)N ((20-60)gf). (tolerance ± 0.0196N (2gf) is allowable), the time for flicking (the duration of the flicking hammer 4 applied force to the cigarette 1) can be maintained for 0.03s (tolerance ± 0.005s is allowed), and the flicking position X2 (the distance X2 from the center force applying point for the flicking hammer 4 applied on the cigarette 1 to the distal end of the cigarette butt) is adjustable within the range of (30-32) mm (tolerance ± 0.5 mm is allowed), and the flicking frequency (the number of times of flicking ashes in one round of the flicking hammer 4 applied force on the cigarette 1, that is, the number of continuous force applying times per round of flicking action) can control the setting to once flicking action and 2 to 4 times of continuous flicking actions(the interval between two flicking action is no more than 1s) per round,

3. The material of the cigarette holder in the clamping device unit is based on the simulated human finger, and its Shore hardness should be 2.7±2.3HA; according to the smoker's flicking habit, the width of the cigarette holder should be 10±0.5mm, and the clamping strength is adjustable within the range of 0.156-0.176N (16-18gf), and ±0.0196N (2gf) of the tolerance is allowed; the clamping position is adjustable within the range of 1-20mm, and the tolerance ±0.5mm is allowed; and the device should also have a control unit for controlling the flicking strength, the time of the flicking applied, the location of the flicking, the timing of the flicking, the clamping strength, and the clamping position and so on.

**Example 1:**

A regular cigarette (brand A) is selected to be a test sample. The invention can be implemented in accordance with the following test condition settings and operational steps:
Place the sample under a constant temperature and humidity environment and adjust the sample according to the requirements of GB/T 16447. The laboratory test atmosphere for the burning cone head falling tendency test shall also be complied with the provisions of GB/T16447.

Step 2: Set the test operational conditions according to the specifications of the cigarette.

The flicking strength and the flicking applied time of the burning cone head falling performance detecting apparatus is set to 0.372±0.0196N (38±2gf) the flicking position of the burning cone head falling performance detecting apparatus is set to 30±0.5mm; the termination mark position of the cigarette burning cone head falling tendency test of the burning cone head falling tendency test device is set to 40±0.5mm; the flicking timing of the burning cone head falling test device is set to that: starting from the second drag of smoking, one round of flicking is applied respectively after every drag, until the termination mark position is reached; the frequency of flicking ash of the burning cone head falling test device is set to that: twice flicking is applied per round, the interval time between two flicking is not more than 1s; the clamping strength of the burning cone head falling test device is set to 0.176±0.0196N (18±2gf); the clamping position of the burning cone head falling tendency test device is set to 18±0.5mm; and the smoking mode of the burning cone head falling tendency test device is set to the ISO3308 standard smoking mode.

Step 3: The adjusted cigarette sample is inserted into the cigarette holder and performs the smoking and flicking actions under the test conditions.

Step 4: When the head falling is happened during the smoking or the termination mark position of the cigarette burning cone head falling tendency test is reached, the test is completed, and the state information of the "falling" and "not falling" of the burning cone of the cigarette and dragging numbers of the occurrence of the head falling are recorded.

Step 5: 40 cigarettes are repeatedly tested and the cigarette burning cone head falling tendency is calculated as a set of test results.

Step 6: Every sample is tested for twice.

Step 7: Calculate the test results.

The test data recording results, presenting the calculation of the cigarette burning cone head falling tendency (HCFP), is calculated according to equation (1):

$$HCFP = \frac{n}{40} \times 100\% \qquad \dots\dots\dots\dots\dots(1)$$

wherein:

HCFP - Cigarette burning cone head falling tendency %;
n - The number of cigarettes having the head falling phenomenon

The number of cigarettes having the head falling phenomenon in two set of 40 cigarettes is 9 and 10.

Step 8: Analyze and determine the results, two testing results are 22.5% and 25%, respectively. The absolute difference of the parallel test results is not more than 20.0%, and the test result is 23.8%.

**Example 2:**

A slim cigarette (brand B) is selected to be a test sample. The invention can be implemented in accordance with the following test condition settings and operational steps:

Place the sample under a constant temperature and humidity environment and adjust the sample according to the requirements of GB/T 16447. The laboratory test atmosphere for the burning cone head falling tendency test shall also be complied with the provisions of GB/T16447.

Step 2: Set the test operational conditions according to the specifications of the cigarette.

The flicking strength and the flicking applied time of the burning cone head falling performance detecting device is set to 0.313±0.0196N (32±2gf); the flicking position of the burning cone head falling performance detecting device is set to 32±0.5mm; the termination mark position of the cigarette burning cone head falling tendency test of the burning cone head falling tendency test device is set to 42±0.5mm; the flicking timing of the burning cone head falling test device is set to that: starting from the second drag of smoking, one round of flicking is applied respectively after every drag, until the termination mark position is reached; the frequency of flicking ash of the burning cone head falling test device is set to that: twice flicking is applied per round, the interval time between two flicking is not more than 1s; the clamping strength of the burning cone head falling test device is set to 0.156±0.196N (16±2gf); the clamping position of the burning cone head falling tendency test device is set to 16±0.5mm; and the smoking mode of the burning cone head falling tendency test device is set to the ISO3308 standard smoking mode.

The adjusted cigarette sample is inserted into the cigarette holder and performs the smoking and flicking actions under the test conditions.

Step 4: When the head falling is happened during the smoking or the termination mark position of the cigarette burning cone head falling tendency test is reached, the test is completed, and the state information of the "falling" and "not falling" of the burning cone of the cigarette and drag numbers of the occurrence of the head falling are recorded.

Step 5: 40 cigarettes are repeatedly tested and the cigarette burning cone head falling tendency is calculated as a set of test results.

Step 6: Every sample is tested for twice.

Step 7: Calculate the test results.

The test data recording results, presenting the calculation of the cigarette burning cone head falling tendency (HCFP), is calculated according to equation (1):

$$HCFP = \frac{n}{40} \times 100\% \qquad \ldots\ldots\ldots\ldots\ldots(1)$$

Wherein:

HCFP - Cigarette burning cone head falling tendency %;
n - The number of cigarettes having the head falling phenomenon.

The number of cigarettes having the head falling phenomenon in two set of 40 cigarettes is 6 and 6.
Step 8: Analyze and determine the results, two testing results are 15% and 15%, respectively. The absolute difference of the parallel test results is not more than 20.0%, and the test result is 15%.

**Claims**

1. An apparatus for assessing hot coal fallout propensity of burning cigarettes based on human behavior features of ash-flicking action, comprising:

   a holding unit for holding a cigarette;
   a suction unit connectable to one end of the cigarette at a contact surface to suck the cigarette;
   a flicking unit disposed adjacent to the holding unit, and the flicking unit being capable of flicking the cigarette; and
   a control unit coupled with the suction unit and the flicking unit, respectively, to control suction and flicking actions, **characterized in that** the flicking unit comprises:

   a flicking arm (3); and
   a flicking hammer (4) disposed at one end of the flicking arm to flick the cigarette under driving of the flicking arm, and an angle (2) between the flicking arm and the cigarette being between 30°-60° when the cigarette is held by the holding unit and when the flicking hammer is in contact with the cigarette, wherein the angle is formed by the center line of the flicking arm and the symmetry plane of the cigarette in the top view when a flicking arm moves to be parallel to a horizontal plane.

2. The apparatus according to claim 1, wherein the holding unit is disposed at a filter tip of the cigarette, and the one end is a free end of the filter tip.

3. The apparatus according to claim 1, wherein the flicking hammer is made of a material with a Mohs hardness in a range of 1-3.

4. The apparatus according to claim 1, wherein a flicking strength of the flicking hammer applied on the cigarette is between 0.196N-0.588N (20gf-60gf); wherein preferably duration for every flicking action applied by the flicking hammer is between 0.025 seconds - 0.035 seconds.

5. The apparatus according to claim 1, wherein a width of a flicking point, where the flicking hammer flicks at the cigarette, is between 9.5mm-10.5mm, and a distance between the flicking point and the contact surface is between 30mm-32mm.

6. The apparatus according to claim 1, wherein the holding unit is made of a material with a Shore hardness in a range of 0.4HA-5.0HA.

7. The apparatus according to claim 1, wherein a holding width of the holding unit holding the cigarette is between 9.5mm-10.5mm, a holding strength of the holding unit is between 0.156N-0.176N (16gf-18gf), and a distance from a holding point of the holding unit to the contact surface is between 18mm-20mm.

8. The apparatus according to claim 1, wherein the control unit is used to control a holding strength of the holding unit, a suction strength and a suction frequency of the suction unit, and a flicking cycle, a position of a flicking point and a flicking strength of the flicking unit.

9. A method for assessing hot coal fallout propensity of burning cigarettes by using the apparatus for assessing hot coal fallout propensity of burning cigarettes according to any one of claims 1 to 8, comprising:

step A: using the holding unit to hold the cigarette and ignite the cigarette;

step B: activating the suction unit by the control unit to suck the cigarette, so as to simulate smoking action of human;

step C: taking k times suction by the suction unit as a cycle, and activating the flicking unit by the control unit to perform a round of flicking actions on the cigarette to simulate human's flicking actions;

step D: stopping detection by the control unit when the cigarette has fallout or the cigarette is burned to a predetermined test termination mark; and

step E: repeating the step B, the step C and the step D for 40 cigarettes, and recording an occurrence number n of fallout so as to calculate hot coal fallout propensity (HCFP) of burning cigarettes by using the following formula:

$$HCFP = n / 40 \times 100\%.$$

10. The method according to claim 9, wherein the step A, the step B, the step C, the step D, and the step E are performed in a constant temperature and humidity environment.

11. The method according to claim 9, wherein a holding width of the holding unit holding the cigarette is between 9.5mm-10.5mm, and a holding strength of the holding unit is between 16gf-18gf, and a distance from a holding point of the holding unit to the one end is between 18mm-20mm.

12. The method according to claim 9, wherein in the step C, the one round of flicking actions comprises performing 1-4 times of flicking actions on the cigarette by using the flicking unit, and an interval time between adjacent two flicking actions is no longer than 1 second.

13. The method according to claim 9, wherein, in the step C, a flicking strength of the flicking hammer applied on the cigarette is between 0.196N-0.588N (20gf-60gf); wherein further preferably, in the step C, a width of a flicking point, where the flicking hammer flicks at the cigarette, is between 9.5mm-10.5mm, and a distance between the flicking point and the one end is between 30mm-32mm.

14. The apparatus according to claim 9, wherein two sets of tests are applied to each cigarette sample, a final HCFP of the cigarette is represented by an average value of the two sets of detection results after the step E, and the detection is performed again when an absolute difference of two sets of detection results is greater than 20%.


**Patentansprüche**

1. Gerät zum Beurteilen der Neigung zum Fallout heißer Glut brennender Zigaretten basierend auf menschlichen Verhaltensmerkmalen der Aschenschnippaktion, aufweisend:

eine Halteeinheit zum Halten einer Zigarette;

eine Saugeinheit, die an ein Ende der Zigarette an einer Kontaktoberfläche zum Saugen an der Zigarette ansteckbar ist;

eine Schnippeinheit, die an die Halteeinheit angrenzend angeordnet ist, wobei die Schnippeinheit dazu geeignet ist, die Zigarette zu schnippen; und

eine Steuereinheit, die mit der Saugeinheit bzw. der Schnippeinheit gekoppelt ist, um die Saug- und die Schnippaktion zu steuern,

**dadurch gekennzeichnet, dass** die Schnippeinheit aufweist:

einen Schnipparm (3); und

einen Schnipphammer (4), der an einem Ende des Schnipparms angeordnet ist, um die Zigarette unter Antreiben des Schnipparms zu schnippen, wobei ein Winkel (2) zwischen dem Schnipparm und der Zigarette zwischen 30° und 60° liegt, wenn die Zigarette von der Halteeinheit gehalten wird, und

wenn der Schnipphammer mit der Zigarette in Kontakt ist, wobei der Winkel durch die Mittenlinie des Schnipparms und die Symmetrieebene der Zigarette in der Draufsicht gebildet ist, wenn sich der Schnipparm bewegt, um zu einer horizontalen Ebene parallel zu sein.

2.  Gerät nach Anspruch 1, wobei die Halteeinheit an einer Filterspitze der Zigarette angeordnet und das eine Ende ein freies Ende der Filterspitze ist.

3.  Gerät nach Anspruch 1, wobei der Schnipphammer aus einem Material mit einer Mohs-Härte in einem Bereich von 1 bis 3 hergestellt ist.

4.  Gerät nach Anspruch 1, wobei eine Schnippkraft des Schnipphammers, die an die Zigarette angelegt wird, zwischen 0,196 N und 0,588 N (20 gf und 60 gf) liegt;
    wobei bevorzugt die Dauer jeder Schnippaktion, die von dem Schnipphammer angewandt wird, zwischen 0,025 Sekunden - 0,035 Sekunden liegt.

5.  Gerät nach Anspruch 1, wobei eine Breite einer Schnippstelle, an der der Schnipphammer an einer der Zigarette schnippt, zwischen 9,5 mm und 10,5 mm liegt, und ein Abstand zwischen der Schnippstelle und der Kontaktoberfläche zwischen 30 mm und 32 mm liegt.

6.  Gerät nach Anspruch 1, wobei die Halteeinheit aus einem Material mit einer Shore-Härte in einem Bereich von 0,4 HA-5,0 HA hergestellt ist.

7.  Gerät nach Anspruch 1, wobei eine Haltebreite der Halteeinheit, die die Zigarette hält, zwischen 9,5 mm und 10,5 mm liegt, eine Haltekraft der Halteeinheit zwischen 0,156 N-0,176 N (16 gf-18 gf) liegt,
    und ein Abstand von einer Haltestelle der Halteeinheit zu der Kontaktoberfläche zwischen 18 mm und 20 mm liegt.

8.  Gerät nach Anspruch 1, wobei die Steuereinheit zum Steuern einer Haltekraft der Halteeinheit, einer Saugkraft und einer Saugfrequenz der Saugeinheit, sowie eines Schnippzyklus, einer Position einer Schnippstelle und einer Schnippkraft der Schnippeinheit verwendet wird.

9.  Verfahren zum Beurteilen einer Neigung zum Fallout heißer Glut Kohle brennender Zigaretten unter Verwendung des Geräts zum Beurteilen der Neigung zum Fallout heißer Glut Kohle brennender Zigaretten nach einem der Ansprüche 1 bis 8, Folgendes umfassend:

    Schritt A: Verwenden der Halteeinheit zum Halten der Zigarette und Anzünden der Zigarette;
    Schritt B: Aktivieren der Saugeinheit durch die Steuereinheit, um an der Zigarette zu saugen, um eine Rauchaktion eines Menschen zu simulieren;
    Schritt C: Betrachten von k-maligem Saugen durch die Saugeinheit als ein Zyklus, und Aktivieren der Schnippeinheit durch die Steuereinheit, um eine Runde von Schnippaktionen an der Zigarette durchzuführen, um die Schnippaktionen eines Menschen zu simulieren;
    Schritt D: Stoppen der Erfassung durch die Steuereinheit, wenn die Zigarette Fallout aufweist oder die Zigarette bis zu einer vorbestimmten Testabschlussmarkierung verbrannt ist; und
    Schritt E: Wiederholen von Schritt B, Schritt C und Schritt D an 40 Zigaretten, und Aufzeichnen einer Auftrittsanzahl n von Fallout, um die Neigung zum Fallout heißer Glut (HCFP) brennender Zigaretten unter Verwendung der folgenden Formel zu berechnen:

$$HCFP = n / 40 \times 100\ \%.$$

10. Verfahren nach Anspruch 9, wobei der Schritt A, der Schritt B, der Schritt C, der Schritt D sowie der Schritt E in einer Umgebung mit konstanter Temperatur und Luftfeuchtigkeit durchgeführt werden.

11. Verfahren nach Anspruch 9, wobei eine Haltebreite der Halteeinheit, die die Zigarette hält, zwischen 9,5 mm und 10,5 mm liegt, und eine Haltekraft der Halteeinheit zwischen 16 gf und 18 gf liegt, und ein Abstand von einer Haltestelle der Halteeinheit zu dem einen Ende zwischen 18 mm und 20 mm liegt.

12. Verfahren nach Anspruch 9, wobei bei Schritt C die eine Runde von Schnippaktionen das 1- bis 4-malige Durchführen von Schnippaktionen an der Zigarette unter Verwendung der Schnippeinheit umfasst, und ein Zeitintervall zwischen aufeinanderfolgenden zwei Schnippaktionen nicht länger als 1 Sekunde ist.

**13.** Verfahren nach Anspruch 9, wobei bei Schritt C eine Schnippkraft des Schnipphammers, die an die Zigarette angelegt wird, zwischen 0,196 N und 0,588 N (20 gf und 60 gf) liegt; wobei ferner bevorzugt bei Schritt C eine Breite einer Schnippstelle, an der der Schnipphammer an der Zigarette schnippt, zwischen 9,5 mm und 10,5 mm liegt, und ein Abstand zwischen der Schnippstelle und dem einen Ende zwischen 30 mm und 32 mm liegt.

**14.** Gerät nach Anspruch 9, wobei zwei Sätze von Tests an jede Zigarettenprobe angewendet werden, eine abschließende HCFP der Zigarette durch einen Mittelwert der zwei Sätze von Erfassungsergebnissen nach dem Schritt E dargestellt ist, und die Erfassung wieder durchgeführt wird, wenn eine Absolutdifferenz von zwei Sätzen von Erfassungsergebnissen größer ist als 20 %.

## Revendications

**1.** Appareil pour évaluer la propension à la chute de charbon chaud de cigarettes en combustion sur la base de caractéristiques comportementales humaines de l'action de frapper pour faire tomber les cendres, comprenant :

une unité de maintien pour maintenir une cigarette ;
une unité d'aspiration pouvant être connectée à une extrémité de la cigarette au niveau d'une surface de contact pour aspirer la cigarette ;
une unité de frappe disposée à côté de l'unité de maintien, l'unité de frappe étant capable de frapper la cigarette ; et
une unité de commande couplée à l'unité d'aspiration et à l'unité de frappe, respectivement, pour commander les actions d'aspiration et de frappe,
**caractérisé en ce que** l'unité de frappe comprend :

un bras de frappe (3) ; et
un marteau de frappe (4)

disposé à une extrémité du bras de frappe pour frapper la cigarette sous l'action du bras de frappe, et un angle (2) entre le bras de frappe et la cigarette étant compris entre 30° et 60° lorsque la cigarette est maintenue par l'unité de maintien, et

lorsque le marteau de frappe est en contact avec la cigarette, l'angle étant formé par la ligne centrale du bras de frappe et le plan de symétrie de la cigarette dans la vue de dessus lorsque le bras de frappe se déplace pour être parallèle à un plan horizontal.

**2.** Appareil selon la revendication 1, dans lequel l'unité de maintien est disposée au niveau de l'embout de filtre de la cigarette, et l'une des extrémités est une extrémité libre de l'embout de filtre.

**3.** Appareil selon la revendication 1, dans lequel le marteau de frappe est fait d'un matériau ayant une dureté Mohs dans une plage allant de 1 à 3.

**4.** Appareil selon la revendication 1, dans lequel une force de frappe du marteau de frappe appliqué sur la cigarette est comprise entre 0,196 N et 0,588 N (20 gf-60 gf) ;
dans lequel, de préférence, la durée de chaque action de frappe appliquée par le marteau de frappe est comprise entre 0,025 seconde et 0,035 seconde.

**5.** Appareil selon la revendication 1, dans lequel une largeur d'un point de frappe où le marteau de frappe frappe la cigarette est comprise entre 9,5 mm et 10,5 mm, et une distance entre le point de frappe et la surface de contact est comprise entre 30 mm et 32 mm.

**6.** Appareil selon la revendication 1, dans lequel l'unité de maintien est faite d'un matériau ayant une dureté Shore dans une plage allant de 0,4 HA à 5,0 HA.

**7.** Appareil selon la revendication 1, dans lequel une largeur de maintien de l'unité de maintien maintenant la cigarette est comprise entre 9,5 mm et 10,5 mm, une force de maintien de l'unité de maintien est comprise entre 0,156 N et 0,176 N (16 gf-18 gf),

et une distance entre un point de maintien de l'unité de maintien et la surface de contact est comprise entre 18 mm et 20 mm.

8. Appareil selon la revendication 1, dans lequel l'unité de commande est utilisée pour commander une force de maintien de l'unité de maintien, une force d'aspiration et une fréquence d'aspiration de l'unité d'aspiration, et un cycle de frappe, une position d'un point de frappe et une force de frappe de l'unité de frappe.

9. Procédé pour évaluer la propension à la chute de charbon chaud de cigarettes en combustion en utilisant l'appareil pour évaluer la propension à la chute de charbon chaud de cigarettes en combustion selon l'une quelconque des revendications 1 à 8, comprenant :

étape A : l'utilisation de l'unité de maintien pour maintenir la cigarette et allumer la cigarette ;
étape B : l'activation de l'unité d'aspiration par l'unité de commande pour aspirer la cigarette, de manière à simuler l'action de fumer d'un être humain ;
étape C : la prise k fois d'aspiration par l'unité d'aspiration comme un cycle, et l'activation de l'unité de frappe par l'unité de commande pour effectuer une série d'actions de frappe sur la cigarette afin de simuler les actions de frappe d'un être humain ;
étape D : l'arrêt de la détection par l'unité de commande lorsque la cigarette est tombée ou lorsque la cigarette est brûlée jusqu'à une marque de fin de test prédéterminée ; et
étape E : la répétition de l'étape B, de l'étape C et de l'étape D pour 40 cigarettes, et l'enregistrement d'un nombre d'occurrences n de chute afin de calculer la propension à la chute de charbon chaud (PCCC) de cigarettes en combustion à l'aide de la formule suivante :

$$PCCC = n / 40 \times 100\ \%.$$

10. Procédé selon la revendication 9, dans lequel l'étape A, l'étape B, l'étape C, l'étape D et l'étape E sont effectuées dans un environnement à température et humidité constantes.

11. Procédé selon la revendication 9, dans lequel une largeur de maintien de l'unité de maintien maintenant la cigarette est comprise entre 9,5 mm et 10,5 mm, et une force de maintien de l'unité de maintien est comprise entre 16 gf et 18 gf, et une distance entre un point de maintien de l'unité de maintien et l'une des extrémités est comprise entre 18 mm et 20 mm.

12. Procédé selon la revendication 9, dans lequel, à l'étape C, la première série d'actions de frappe consiste à effectuer 1 à 4 fois des actions de frappe sur la cigarette en utilisant l'unité de frappe, et un intervalle de temps entre deux actions de frappe adjacentes n'est pas supérieur à 1 seconde.

13. Procédé selon la revendication 9, dans lequel, à l'étape C, la force de frappe du marteau de frappe appliqué sur la cigarette est comprise entre 0,196 N et 0,588 N (20 gf-60 gf) ;
de manière davantage préférée, à l'étape C, une largeur d'un point de frappe, où le marteau de frappe frappe la cigarette, est comprise entre 9,5 mm et 10,5 mm, et la distance entre le point de frappe et l'une des extrémités est comprise entre 30 mm et 32 mm.

14. Appareil selon la revendication 9, dans lequel deux séries de tests sont appliquées à chaque échantillon de cigarette, une PCCC finale de la cigarette est représentée par une valeur moyenne des deux séries de résultats de détection après l'étape E, et la détection est effectuée à nouveau lorsqu'une différence absolue de deux séries de résultats de détection est supérieure à 20 %.

Fig. 1

Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 102937639 A
- CN 204165850 U
- CN 201310227468X
- CN 201510973214
- CN 105571960 A

### Non-patent literature cited in the description

- **PEACE.** World No Tobacco Day: Interpretation of the cigarette butt [J]. *China Fire,* 2011, vol. 11, 38-42
- **WANG HAIBIN ; LIU DEHU ; WU ZHAOGANG et al.** Analysis and research on the burning phenomenon of cigarette burning end [A]. *China Tobacco Society 2010 Symposium Set,* 2010, 285-287